# EUROPEAN PATENT SPECIFICATION

(11) **EP 4 086 343 B1**
(45) Date of publication and mention of the grant of the patent: **19.08.2026**
(21) Application number: 21738086.4
(22) Date of filing: 06.01.2021
(51) Int. Cl.: C12N 9/04, C12P 7/64, C12N 15/62

(54) **USE OF BIOLOGICAL ENZYME FOR PREPARING ORLISTAT INTERMEDIATE, AND PREPARATION METHOD**
VERWENDUNG VON BIOLOGISCHEM ENZYM ZUR HERSTELLUNG EINES ORLISTATZWISCHENPRODUKTS UND VERFAHREN ZUR HERSTELLUNG
UTILISATION D'UNE ENZYME BIOLOGIQUE POUR LA PRÉPARATION D'UN INTERMÉDIAIRE D'ORLISTAT ET PROCÉDÉ DE PRÉPARATION

(30) Priority: 07.01.2020 CN 202010015510
(43) Date of publication of application: 09.11.2022
(73) Proprietor: ZEIN BIOTECHNOLOGY CO., LTD., Chongqing 400039 (CN)
(72) Inventor: XU, Tianshuai, Chongqing 400039 (CN); GONG, Dayong, Chongqing 400039 (CN); XIAO, Yumei, Chongqing 400039 (CN); WANG, Zhanghong, Chongqing 400039 (CN); HUANG, Zhichuan, Chongqing 400039 (CN); HUANG, Shan, Chongqing 400039 (CN); ZHANG, Lei, Chongqing 400039 (CN); GAO, Xin, Chongqing 400039 (CN); SHEN, Junwei, Chongqing 400039 (CN)
(74) Representative: Lorenz Seidler Gossel Part. mbB
(86) International application number: PCT/CN2021/070495
(87) International publication number: WO 2021/139689

(56) References cited:
- WO-A2-2007/029086
- CN-A- 101 348 475
- CN-A- 102 976 940
- CN-A- 107 058 251
- CN-A- 108 484 536
- CN-A- 109 022 473
- CN-A- 111 154 736
- CN-B- 102 634 546
- DATABASE UniProt [online] 16 October 2019 (2019-10-16), "SubName: Full=Diacetyl reductase [(S)-acetoin forming] {ECO:0000313|EMBL:QDU26184.1}; EC=1.1.1.304 {ECO:0000313|EMBL:QDU26184.1};", XP002811451, retrieved from EBI accession no. UNIPROT:A0A517Y7G4 Database accession no. A0A517Y7G4
- DATABASE UniProt [online] 18 July 2018 (2018-07-18), "SubName: Full=NAD(P)-dependent dehydrogenase (Short-subunit alcohol dehydrogenase family) {ECO:0000313|EMBL:PUB44836.1};", XP002811452, retrieved from EBI accession no. UNIPROT:A0A2T6MC21 Database accession no. A0A2T6MC21
- DATABASE PROTEIN 20 June 2019 (2019-06-20), ANONYMOUS: "glucose 1-dehydrogenase [Singulisphaera acidiphila]", XP055827462, retrieved from GENBANK Database accession no. WP_015245403
- DATABASE PROTEIN 18 June 2019 (2019-06-18), GENBANK ACCESSION NUMBER: WP_010403640.1: "glucose 1-dehydrogenase [Singulisphaera echinoides]", XP055827471, retrieved from GENBANK Database accession no. WP_010403640
- DATABASE PROEIN 3 December 2013 (2013-12-03), GENBANK ACCESSION NUMBER: EGU12837: "Proteophosphoglycan ppg4 [Rhodotorula toruloides ATCC 204091]", XP055827472, retrieved from GENBANK Database accession no. EGU12837
- XU, QINYAO ET AL.: "A New Route for the Preparation of Orlistat", CHINESE JOURNAL OF ORGANIC CHEMISTRY, vol. 30, no. 8, 31 August 2010 (2010-08-31), pages 1175 - 1179, XP055827473, ISSN: 0253-2786
- BAI TINGLI, ZHANG DAOZHONG, LIN SHUANGJUN, LONG QINGSHAN, WANG YEMIN, OU HONGYU, KANG QIANJIN, DENG ZIXIN, LIU WEN, TAO MEIFENG: "Operon for Biosynthesis of Lipstatin, the Beta-Lactone Inhibitor of Human Pancreatic Lipase", APPLIED AND ENVIRONMENTAL MICROBIOLOGY, vol. 80, no. 24, 15 December 2014 (2014-12-15), US, pages 7473 - 7483, XP055827474, ISSN: 0099-2240, DOI: 10.1128/AEM.01765-14

## Description

### FIELD

The present invention relates to the technical field of biopharmaceuticals and biochemical industries, and in particular to a method for the biosynthesis of (R)-β-hydroxytetradecanoate compounds.

### BACKGROUND

Obesity treatment drugs are mainly divided into two categories, i.e. central nervous system-acting weight loss drugs and non-central nervous system-acting weight loss drugs. The weight loss effect of the central nervous system-acting weight loss drugs is obvious, but the side effects are relatively great, and the clinical application is greatly limited. Among them, fenfluramine was withdrawn from the market in 1997 because it can cause pulmonary hypertension and hypertrophic heart valve disease. Sibutramine was discontinued for production, sale and use in 2010 by the China Food and Drug Administration due to its potential to increase serious cardiovascular risks. Locaserin (trade name Belviq) was approved by the FDA in June 2012, but it still has the risk of increasing heart valve disease and adverse cardiovascular events, and the safety of clinical application needs to be further verified.

Orlistat is the first non-central nervous-acting weight loss drug, and is currently the only OTC weight loss drug in the world. Since its launch in 1998, it has become the drug of choice for weight loss treatment due to its definite efficacy and high safety. Orlistat acts directly on lipase in the gastrointestinal tract, and has a significant effect on inhibiting fat absorption. Its target is highly specific, and it has no effect on other gastrointestinal enzymes, and does not require systemic absorption to exert its drug effect. Its systemic drug exposure in vivo is very low, and the main side effect is gastrointestinal reactions, and the safety is excellent. Orlistat has been sold globally for more than 20 years, and has been approved for marketing in more than 145 countries. Its excellent efficacy and safety have been verified by a large number of clinical applications.

The market demand of orlistat is large, and it is extremely important to find efficient synthetic methods of orlistat and its intermediates. (R)-β-hydroxytetradecanoate is an important intermediate in the synthesis of the drug orlistat, and the optical purity of this intermediate is extremely critical. How to efficiently obtain optically pure (R)-β-hydroxytetradecanoate?

For example, Chinese Patent CN101538285B reported a prepared (R)-metal catalyst [(R)-Ru(MeOBIPHEP)Cl2]2•NEt3 to catalyze asymmetric hydrogenation, but this chemical catalytic reaction can only obtain the product with ee value>98.5% by being performed under the conditions of strong acid and 60bar high pressure. The disadvantages of such methods are: 1) expensive (R)-ligand and precious metal catalysts are required; 2) the catalysts need to be prepared for immediate use, and the process stability is difficult to be guaranteed; 3) high-pressure hydrogenation equipment is required; 4) acid-resistant equipment is required; 5) poor reaction control will affect the ee value.

CN 102 634 546 B discloses an enzymatic method for the manufacture of an ornistat intermediate by applying a ketoreductase in combination with a dehydrogenase. CN 102 058 251 A as well as the EBI database (Accession nos. A0A517Y7G4 and A0A2T6MC21) discloses biological enzymes of respective activities.

### SUMMARY

In view of the above-mentioned shortcomings of the prior art, the inventors of the present invention carried out a systematic improvement by using a biological enzyme method to achieve the large-scale production of key intermediate of orlistat.

In view of this, the first object of the present invention is to provide a new use of a biological enzyme in the preparation of the orlistat intermediate. The biological enzyme can effectively act on the substrate β-carbonyltetradecanoate to prepare the orlistat intermediate with high purity.

To achieve the above object, the technical solution of the present invention is:
use of a biological enzyme in the preparation of the orlistat intermediate (final product), wherein the substrate for the biological enzyme is β-carbonyltetradecanoate (raw material), and the structural formula is as shown in I; the orlistat intermediate is (R)-β-hydroxytetradecanoate, and the structural formula is as shown in II; R in the structural formula I refers to a saturated alkyl group containing 1-3 carbon atoms, and R in the structural formula II refers to a saturated alkyl group containing 1-3 carbon atoms.

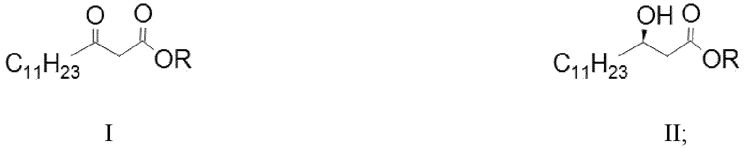

The biological enzyme is ketoreductase, and the amino acid sequence of the biological enzyme is as shown in SEQ ID NO: 1, which is derived from Singulisphaera acidiphila, encoded as WP_015245403.1 in the NCBI database, belonging to the short-chain dehydrogenase family; and/or as shown in SEQ ID NO: 2, which is derived from Sphingomonas echinoides, encoded as WP_010403640.1 in the NCBI database, belonging to the short-chain dehydrogenase family; and/or as shown in SEQ ID NO: 4, which is derived from Rhodotorula toruloides, and is a truncated protein of the protein encoded as EGU12837.1 in the NCBI database, belonging to the short-chain dehydrogenase family. Or the biological enzyme is a fusion enzyme of ketoreductase and glucose dehydrogenase, and the amino acid sequence of the fusion enzyme is as shown in SEQ ID NO:8 and/or as shown in SEQ ID NO:9. The inventors of the present invention have surprisingly found that the natural or genetically/protein-engineered biological enzyme has a good catalytic effect on the substrate, and the final product with high optical purity is obtained.

Further, the biological enzyme having SEQ ID NO:1 and/or SEQ ID NO:2, SEQ ID NO:4, and SEQ ID NO:8 and/ or SEQ ID NO: 9 can also be used to prepare the orlistat intermediate.

Further, in the fusion enzyme of ketoreductase and glucose dehydrogenase, the ketoreductase and the glucose dehydrogenase are connected by a linker;
further, the amino acid sequence of the linker is as shown in SEQ ID NO:5.

Further, the fusion enzyme of ketoreductase and glucose dehydrogenase is ketoreductase-linker-glucose dehydrogenase, or glucose dehydrogenase-linker-ketoreductase; optionally, the amino acid sequence of the glucose dehydrogenase is as shown in SEQ ID NO:3.

Further, any one of the above-mentioned biological enzymes is enzyme powder and/or enzyme solution and/or immobilized enzyme.

Further, R in the structural formula I is any one of methyl, ethyl, n-propyl or isopropyl, and R in the structural formula II is any one of methyl, ethyl, n-propyl or isopropyl.

The second object of the present invention is to provide a fusion enzyme, and the fusion enzyme is a fusion enzyme of ketoreductase and glucose dehydrogenase, and the amino acid sequence of the fusion enzyme is as shown in SEQ ID NO:8 and/or SEQ ID NO: 9.

Further, the ketoreductase and the glucose dehydrogenase in the fusion enzyme are connected by a linker; optionally, the amino acid sequence of the linker is as shown in SEQ ID NO:5.

Further, the fusion enzyme is ketoreductase-linker-glucose dehydrogenase, or glucose dehydrogenase-linker-ketoreductase;
optionally, the amino acid sequence of the ketoreductase is as shown in SEQ ID NO: 1 and/or as shown in SEQ ID NO: 2, and/or as shown in SEQ ID NO: 4;
optionally, the amino acid sequence of the glucose dehydrogenase is as shown in SEQ ID NO:3.

The third object of the present invention is to provide a method for encoding the nucleotide sequences of any of the above-mentioned fusion enzymes and a construction method thereof.

The nucleotide sequence of the fusion enzyme is as shown in SEQ ID NO:6 or SEQ ID NO:7.

Further, the construction method of the nucleotide sequence comprises: inserting the linker sequence shown in SEQ ID NO:5 at the 3' end of the gene fragment of glucose dehydrogenase shown in SEQ ID NO:3, and then connecting the gene fragment of ketoreductase shown in SEQ ID NO:4 to form a recombinant plasmid with the nucleotide sequence shown in SEQ ID NO:6.

Further, the nucleotide sequence of the fusion enzyme SEQ ID NO: 6 is connected with the carrier pET28a through the restriction enzyme sites NdeI and XhoI at both ends to form a double-enzyme fusion expression plasmid pET28a-G3790, which is then transferred to Escherichia coli for screening, inoculation and culturing to obtain bacterial cells; the bacterial cells are broken and centrifuged to obtain crude enzyme solution, and the crude enzyme solution is freeze-dried to obtain enzyme powder.

Further, the construction method of the nucleotide sequence comprises: inserting the linker sequence shown in SEQ ID NO:5 at the 3' end of the gene fragment of ketoreductase shown in SEQ ID NO:4, and then connecting the gene fragment of glucose dehydrogenase shown in SEQ ID NO:3 to form the nucleotide sequence shown in SEQ ID NO:7.

Further, the nucleotide sequence of the fusion enzyme SEQ ID NO: 7 is connected with the carrier pET28a through the restriction sites NdeI and XhoI at both ends to form a double-enzyme fusion expression plasmid pET28a-G3790, which is then transferred to Escherichia coli for screening, inoculation, and culturing to obtain bacterial cells; the bacterial cells are broken and centrifuged to obtain crude enzyme solution, and the crude enzyme solution is freeze-dried to obtain enzyme powder.

The fourth object of the present invention is to provide a composition, in which the two can form a synergistic relationship between an enzyme and a substrate, and a product as shown in structural formula II can be obtained.

To achieve the above object, the technical solution of the present invention is:
a composition comprising any of the biological enzymes described above and a substrate, the substrate is β-carbonyltetradecanoate. The structural formula of the β-carbonyltetradecanoate is as shown in formula I; the biological enzyme is ketoreductase or a fusion enzyme of ketoreductase and glucose dehydrogenase, and the amino acid sequence of the ketoreductase is as shown in SEQ ID NO: 1 and/or as shown in SEQ ID NO:2, and/or as shown in SEQ ID NO:4; the amino acid sequence of the fusion enzyme is as shown in SEQ ID NO:8 and/or as shown in SEQ ID NO:9; 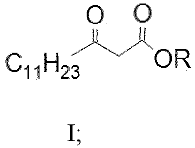
R in structural formula I refers to a saturated alkyl group containing 1-3 carbon atoms.

Further, the ketoreductase and the glucose dehydrogenase in the fusion enzyme are connected by a linker; optionally, the amino acid sequence of the linker is as shown in SEQ ID NO:5.

Further, the fusion enzyme is ketoreductase-linker-glucose dehydrogenase, or glucose dehydrogenase-linker-ketoreductase.

Further, the amino acid sequence of the glucose dehydrogenase is as shown in SEQ ID NO:3.

Further, the biological enzyme is enzyme powder and/or enzyme solution and/or immobilized enzyme.

Further, R in the structural formula I is any one of methyl, ethyl, n-propyl or isopropyl, and R in the structural formula II is any one of methyl, ethyl, n-propyl or isopropyl.

Further, in the composition, the weight ratio of the biological enzyme to the substrate is 1:1.1-150.

Preferably, in the composition, the weight ratio of the biological enzyme to the substrate is 1:20, 1:30, 1:40, 1:50, 1:60, 1:70, 1:80, 1:90, 1:100, 1:110, 1:120, 1:130, 1:140 and/or 1:150.

The fifth object of the present invention is to provide a reaction system for preparing (R)-β-hydroxytetradecanoate. (R)-β-hydroxytetradecanoate can be obtained through this reaction system without resorting to severe conditions of high temperature and high pressure.

To achieve the above object, the technical solution of the present invention is:
the reaction system is composed of the substrate shown in I, biological enzyme, glucose, glucose dehydrogenase, NADP⁺ and buffer solution; the biological enzyme described here is ketoreductase, and the amino acid sequence of the ketoreductase is as shown in SEQ ID NO: 1 and/or as shown in SEQ ID NO: 2, and/or as shown in SEQ ID NO: 4; or the reaction system is composed of the substrate shown in I, biological enzyme, glucose, NADP⁺ and buffe solutionr; wherein, the biological enzyme is a fusion enzyme of ketoreductase and glucose dehydrogenase, and the amino acid sequence of the ketoreductase is as shown in SEQ ID NO: 1 and/or as shown in SEQ ID NO:2, and/or as shown in SEQ ID NO: 4; the amino acid sequence of the fusion enzyme of ketoreductase and glucose dehydrogenase is as shown in SEQ ID NO:8 and/or SEQ ID NO:9; 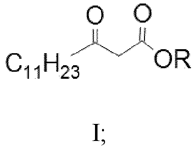
R in the structural formula I refers to a saturated alkyl group containing 1-3 carbon atoms.

Further, the amino acid sequence of the glucose dehydrogenase is as shown in SEQ ID NO:3. Further, the ketoreductase and the glucose dehydrogenase in the fusion enzyme are connected by a linker; optionally, the amino acid sequence of the linker is as shown in SEQ ID NO:5.

Further, the fusion enzyme is ketoreductase-linker-glucose dehydrogenase, or glucose dehydrogenase-linker-ketoreductase.

Further, the amino acid sequence of the glucose dehydrogenase is as shown in SEQ ID NO:3.

Further, the biological enzyme is enzyme powder and/or enzyme solution and/or immobilized enzyme.

Further, R in the structural formula I is any one of methyl, ethyl, n-propyl or isopropyl, and R in the structural formula II is any one of methyl, ethyl, n-propyl or isopropyl.

Further, the pH value of the reaction system is 6.0-8.0.

Further, the buffer solution in the reaction system is PBS buffer or Tris-HCl buffer.

Further, the concentration of the substrate in the reaction system is 20g/L-150g/L.

Further, in the reaction system, the concentration of the NADP⁺ is 0.1-0.5 g/L.

Further, in the reaction system, the molar ratio of the compound represented by the structural formula I to the glucose is 1:(1.2-4).

Further, in the reaction system, the concentration of the buffer solution is 0.01-0.5 mol/L.

Further, the reaction time of the reaction system is not more than 15 hours to obtain the reaction solution.

The sixth object of the present invention is to provide a preparation method of the orlistat intermediate, which is suitable for industrialized large-scale production.

The biosynthesis method of (R)-β-hydroxytetradecanoate, the reaction formula is as follows: 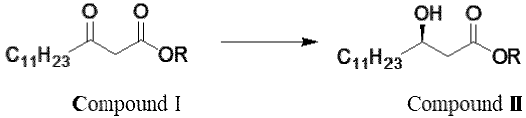
R in compound I and compound II refers to a saturated alkyl group containing 1-3 carbon atoms, preferably, methyl, ethyl, n-propyl or isopropyl;
the biosynthesis method comprises reacting compound I in ketoreductase, glucose, glucose dehydrogenase and NADP⁺, or in the fusion enzyme of ketoreductase and glucose dehydrogenase, glucose and NADP⁺ to obtain compound II .

In embodiments of the present invention, the biosynthesis method comprises the following steps: taking compound I and placing it in a buffer solution, and then adding ketoreductase or a fusion enzyme of ketoreductase and glucose dehydrogenase, glucose, glucose dehydrogenase (glucose dehydrogenase does not need to be added when the fusion enzyme of ketoreductase and glucose dehydrogenase is added) and NADP⁺ to obtain a mixed solution, stirring and reacting the mixed solution at 20-40°C, during the whole process of which the pH is adjusted by NaOH aqueous solution, monitoring the conversion rate of the reaction by liquid chromatography, until the reaction is completed when the conversion rate is more than 99%, then adding extraction solvent for extraction, and combining the organic phases, concentrating under reduced pressure, and cooling for crystallization, separating out the product to obtain a white solid, i.e. compound II, and optionally, performing further recrystallization with n-hexane to obtain a product with higher purity.

In some embodiments of the present invention, the concentration of the aqueous NaOH solution can be 2M.

In some embodiments of the present invention, the buffer solution has a pH value of 6.0-8.0; preferably, the buffer solution is PBS (i.e. phosphate) buffer or Tris-HCl buffer; more preferably, the concentration of the buffer solution is 0.01-0.5mol/L PBS phosphate buffer or 0.01-0.5mol/L Tris-HCl buffer.

In embodiments of the present invention, the pH range is controlled at 7.0-7.5 during the reaction process.

In embodiments of the present invention, the amino acid sequence of the ketoreductase is as shown in SEQ ID NO: 1, SEQ ID NO: 2 and SEQ ID NO: 4 of the present application; the amino acid sequence of the fusion enzyme of ketoreductase and glucose dehydrogenase is as shown in SEQ ID NO:8 and/or as shown in SEQ ID NO:9; the form of the ketoreductase or the fusion enzyme of ketoreductase and glucose dehydrogenase can be enzyme powder, enzyme solution, or immobilized enzyme; the amino acid sequence of the glucose dehydrogenase is as shown in SEQ ID NO: 3 of the present application, and the form of the glucose dehydrogenase can be enzyme powder, enzyme solution, or immobilized enzyme.

In embodiments of the present invention, the NADP⁺ refers to nicotinamide adenine dinucleotide phosphate, i.e. the oxidized form of reduced coenzyme II (NADPH); the concentration of NADP⁺ in the mixed solution is 0.1-0.5 g/ L.

In embodiments of the present invention, the concentration of compound I in the mixed solution is 20 g/L-150 g/L.

In embodiments of the present invention, the molar ratio of compound I to glucose in the mixed solution is 1:(1.2-4).

In some embodiments of the present invention, preferably, the temperature of the reaction is maintained at 35°C.

In some embodiments of the present invention, the extraction solvent is used for extraction twice; the extraction solvent is anhydrous ethanol or ethyl acetate.

The technical solution for the above content is summarized, specifically:
a method for preparing the orlistat intermediate, comprising reacting the reaction system, wherein the reaction system is reacted at 20-40°C with stirring to obtain a reaction solution of the orlistat intermediate. The intermediate of the orlistat intermediate is (R)-β-hydroxytetradecanoate, and the structural formula is as shown in II.

Further, in the method, an aqueous NaOH solution is used as a pH adjusting agent.

Further, in the method, the reaction time does not exceed 15 hours.

Further, in the method, the orlistat intermediate is extracted with a solvent from the obtained reaction solution.

Further, in the method, the solvent is anhydrous ethanol or ethyl acetate.

Further, in the method, the obtained extract is concentrated under reduced pressure, and cooled for crystalization to obtain a white crystalline solid, which is compound II.

Orlistat prepared from the orlistat intermediate.

**The beneficial effects of the present invention are:**
the biological enzyme used in the present invention can tolerate a substrate concentration of up to 150 g/L, and the enzyme activity is not inhibited by the substrate or product.

The method for preparing the orlistat intermediate of the present invention is a biological enzyme method, and the method has mild conditions, simple equipment requirements, and simple operation, and the method generates less waste water, waste residue, and waste gas, and has no heavy metal pollution, and is environmentally friendly and conducive to industrial production..

The conversion rate of the enzyme catalysis process in the present invention is as high as 99% or more, and the chiral ee value can reach more than 99%. The reaction can be completed within 15 hours, the product concentration is high, and the substrate is almost completely converted, which can simplify the post-processing steps of the reaction solution. A product with high purity can be obtained only through a simple extraction and crystallization step, which greatly reduces the production cost.

### BRIEF DESCRIPTION OF THE DRAWINGS

FIG. 1 is the HPLC chromatogram of the conversion rate measured in example 5.
Fig. 2 is the HPLC spectrum of product purity in example 5.
Fig. 3 is the HPLC chromatogram of product chiral purity in example 5.
Figure 4 is the plasmid map of the fusion enzyme G3790 expression plasmid.
Figure 5 is the plasmid map of the fusion enzyme 3790G expression plasmid.
Figure 6 is the G3790 fusion enzyme protein band.
Figure 7 is the 3790G fusion enzyme protein band.

### DETAILED DESCRIPTION OF EMBODIMENTS

The embodiments are given to better illustrate the present invention.

The present invention relates to the reduction of β-carbonyltetradecanoate to (R)-β-hydroxytetradecanoate under the catalysis of the biological enzyme. The reaction formula is as follows:

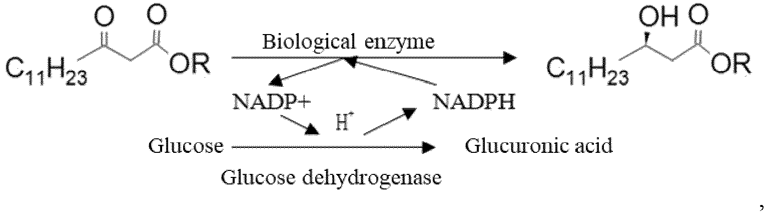

β-carbonyltetradecanoate, as a substrate, is catalyzed by a certain enzyme in an environment containing NADP⁺ coenzyme, glucose and glucose dehydrogenase, to obtain (R)-β-hydroxytetradecanoate with extremely high chiral purity; wherein the catalytic enzyme generally belongs to the short-chain dehydrogenase family, such as different types of ketoreductases in the embodiments of the present invention; it can also be a fusion enzyme of different enzymes, such as the fusion enzymes of reductase and glucose dehydrogenase in some embodiments of the present invention; wherein, glucose is dehydrogenated under the action of glucose dehydrogenase to obtain H⁺, and then NADP⁺ coenzyme carries H⁺ and participates in the reduction reaction of β-carbonyltetradecanoate to obtain β-hydroxytetradecanoate.

The ketoreductase JR3789 of the present invention is derived from Singulisphaera acidiphila and encoded WP_015245403.1 in the NCBI database, belonging to the short-chain dehydrogenases family. The amino acid sequence of the ketoreductase is as shown in SEQ ID NO:1, and the size is 249 amino acids.
Amino acid sequence SEQ ID NO: 1:

“MGKLDNKVAVITGGNSGMGLATAQRFVSEGAYVFITGRRQAELDKAVDLIG
KNVTGVQGDVSNLADLDRLYATVKEQKGRVDVLFANAGVGELAPLGSITEEQFDKVFNIN
VRGLLFTVQKALPLFQDGGSIILNASIASIKGMPAFSVYSASKAAVRSFARSWTVDLKGRKIR
INTLSPGPIDTPILSGLASTEEELKQVKADLAAQVPLGRMGTSDEIANVALFLASDDSSYVTG
IELFVDGGMAQI”.

The ketoreductase JR37150 of the present invention is derived from Sphingomonas echinoides and encoded WP_010403640.1in the NCBI database, belonging to the short-chain dehydrogenases family. The amino acid sequence of the ketoreductase is as shown in SEQ ID NO: 2, and the size is 259 amino acids.
Amino acid sequence SEQ ID. NO: 2:

“MARLAGKVALVTGGASVPGLGSATAIRFAQEGATVYLTDRDLAGAQAVAAQ
ITAAGGRATALEHDVTSEADWDRVLAAIDAAEGRLDILVNNAGIAVLGPLEDVTAADFLRQ
NDVNLNSVFHGSKRALVMMRRPGDGGTARGGSIINISSVAGLIGVPGCGSYAASKGGVRLF
SKVVALEGAADGVRCNSVHPGMIATNIQGVALEDNAANFDAVMALIPMVRMGEPEDIANM
NLFLASDESRYITGAEFVVDGGMTAR”.

The glucose dehydrogenase GDH of the present invention is derived from Bacillus subtilis QB928 and encoded AFQ56330.1in the NCBI database, belonging to the short-chain dehydrogenases family. The amino acid sequence of the dehydrogenase is as shown in SEQ ID NO:3, and the size is 263 amino acids.
Amino acid sequence SEQ ID NO:3:

“MYMYPDLKGKVVAITGAASGLGKAMAIRFGKEQAKVVINYYSNKQDPNE
VKEEVIKAGGEAVVVQGDVTKEEDVKNIVQTAIKEFGTLDIMINNAGLENPVPSHEMPLKD
WDKVIGTNLTGAFLGSREAIKYFVENDIKGNVINMSSVHEVIPWPLFVHYAASKGGIKLMT
ETLALEYAPKGIRVNNIGPGAINTPINAEKFADPKQKADVESMIPMGYIGEPEEIAAVAAWLA
SKEASYVTGITLFADGGMTQYPSFQAGRG”.

The ketoreductase JR3790 of the present invention is derived from Rhodotorula toruloides, and is a truncated protein (position 70-317) of the protein encoded EGU12837.1 in the NCBI database, belonging to the short-chain dehydrogenases family. The amino acid sequence of the ketoreductase is as shown in SEQ ID NO:4, and the size is 248 amino acids.
Amino acid sequence SEQ ID NO: 4: 
“MSSPAPTVYVISGASRGIGFAITSILAQHDNVLIFAGARDLKSAQLNELAQKS
SGKVIPVKLESTSVEDAAALAKVVEEKAGKVDYVLAVAGISQSTDPIAQVSLDDVRRHFEV
NTIGPLVLFQALLPLTTKSTAPHFIVVSTIAGSIASMPQVTFPVSAYAISKTAVNSAVGRIAIEHP
DLDAFVCHPGFVSSDMVKQFAEKTGAPLSDFESFGMITPEESAASLVKLFDEAKKETHSGK
FFNVDGT”.
Linker sequence SEQ ID NO: 5:

“EFEEEEKKKQQEEEAERLRRIQEEMEKERKRREEDEERRRKEEEERRMKLE
MEAKRKQEEEERKKREDDEKRKKKL”.

HPLC detection conditions: OD-H chromatographic column; mobile phase is n-hexane: isopropanol=98:2; flow rate 1.0mL /min. The specification of the detection instrument is: DAD1C, Sig=210, 4 Ref=360,100.

The biological enzymes (SEQ ID NO: 1, SEQ ID NO:2) and glucose dehydrogenase (SEQ ID NO:3) are synthesized by Nanjing GenScript Biotechnology Co., Ltd., and are commercialized.

### Example 1

1g of methyl β-carbonyltetradecanoate and 1.5g of glucose were weighed and placed in a 100mL three-necked flask, then added with 50mL of PBS buffer with a concentration of 50mM and a pH value of 7.0. The three-necked flask was put into a constant temperature water bath, in which the stirring speed was adjusted to 800rpm, and the temperature was 35°C, and then 10mg NADP⁺, 25mg glucose dehydrogenase GDH enzyme powder (shown in SEQ ID NO: 3), and 100mg ketoreductase JR3789 enzyme powder (shown in SEQ ID NO: 1) were added respectively to obtain a mixed reaction solution. The pH was adjusted and maintained at 7.0-7.5 using 2M NaOH solution, the temperature was maintained at 35°C, and the reaction progress was monitored by HPLC. The reaction was completed in 9h, and the conversion rate was measured as >99%

After the reaction was completed, the obtained solution was heated to a temperature of 60°C and maintained for 15min, then cooled to 20-25°C, and added with 80mL of ethyl acetate for extraction with stirring for 20min, and filtered. The organic phase was taken after the filtrate was layered. The aqueous phase was extracted once more with 50mL of ethyl acetate, the layers were separated, and the organic phase was taken. The organic phases were combined, concentrated under reduced pressure, and then slowly cooled to separate out the product. A crude product with a purity of 98.10% and an optical purity of 98.45% was obtained. The crude product was added with 2 times the volume of n-hexane, heated, dissolved, and cooled for crystalization. The crystals were collected and air-dried at room temperature to obtain 0.89g of a white crystalline product with a measured purity of 99.99%, an ee value of 99.91%, and a total yield of 89%.

### Example 2

1g of ethyl β-carbonyltetradecanoate and 1.5g of glucose were weighed and placed in a 100mL three-necked flask, then added with 50mL of PBS buffer with a concentration of 50mM and a pH value of 7.0. The three-necked flask was put into a constant temperature water bath, in which the stirring speed was adjusted to 900rpm, and the temperature was 35°C, and then 10mg NADP⁺, 35mg glucose dehydrogenase GDH enzyme powder (shown in SEQ ID NO: 3), and 150mg ketoreductase JR3789 enzyme powder (shown in SEQ ID NO: 1) were added respectively to obtain a mixed reaction solution. The pH was adjusted and maintained at 7.0-7.5 using 2M NaOH solution, the temperature was maintained at 35°C, and the reaction progress was monitored by HPLC. The reaction was completed in 10h, and the conversion rate was measured as >99%.

After the reaction was completed, the obtained solution was heated to a temperature of 60°C and maintained for 15min, then cooled to 20-25°C, and added with 80mL of ethyl acetate for extraction with stirring for 20min, and filtered. The organic phase was taken after the filtrate was layered. The aqueous phase was extracted once more with 50mL of ethyl acetate, the layers were separated, and the organic phase was taken. The organic phases were combined, concentrated under reduced pressure, and then slowly cooled to separate out the product. 0.84g of a white product with a purity of 98.42%, an ee value of 98.15%, and a total yield of 84% was obtained.

### Example 3

5g of methyl β-carbonyltetradecanoate and 7.5g of glucose were weighed and placed in a 100mL three-necked flask, and then added with 50mL of PBS buffer with a concentration of 50mM and a pH value of 7.0. The three-necked flask was put into a constant temperature water bath, in which the stirring speed was adjusted to 800rpm, and the temperature was 35°C, and then 50mg NADP⁺, 125mg glucose dehydrogenase GDH enzyme powder (shown in SEQ ID NO:3), and 500mg ketoreductase JR3789 enzyme powder (shown in SEQ ID NO: 1) were added respectively to obtain a mixed reaction solution. The pH was adjusted and maintained at 7.0-7.5 using 2M NaOH solution, and the temperature was maintained at 35 °C. The reaction progress was monitored by HPLC. The reaction was completed in 13 h, and the conversion rate was measured as >99%.

After the reaction, the obtained solution was heated to a temperature of 60°C and maintained for 15min, then cooled to 20-25°C, and added with 80mL of ethyl acetate for extraction with stirring for 20min, and filtered. The organic phase was taken after the filtrate was layered. The aqueous phase was extracted once more with 50mL of ethyl acetate, the layers were separated, and the organic phase was taken. The organic phases were combined, concentrated under reduced pressure, and then slowly cooled to separate out the product. 4.65g of a white product with a purity of 98.12%, an ee value of 98.45%, and a total yield of 93% was obtained.

### Example 4

5g of methyl β-carbonyltetradecanoate and 7.5g of glucose were weighed and placed in a 100mL three-necked flask, and then added with 50mL of PBS buffer with a concentration of 50mM and a pH value of 7.0. The three-necked flask was put into a constant temperature water bath, in which the stirring speed was adjusted to 800rpm, and the temperature was 35°C, and then 50mg NADP⁺, 300mg glucose dehydrogenase GDH enzyme powder (shown in SEQ ID NO: 3), and 800mg ketoreductase JR37150 enzyme powder (shown in SEQ ID NO:2) were added respectively to obtain a mixed reaction solution. The pH was adjusted and maintained at 7.0-7.5 using 2M NaOH solution, and the temperature was maintained at 35 °C. The reaction progress was monitored by HPLC. The reaction was completed in 35h, and the conversion rate was measured as >99%.

After the reaction, the obtained solution was heated to a temperature of 60°C and maintained for 15min, then cooled to 20-25°C, and added with 100mL of ethyl acetate for extraction with stirring for 20min, and filtered. The organic phase was taken after the filtrate was layered. The aqueous phase was extracted once more with 60mL of ethyl acetate, the layers were separated, and the organic phase was taken. The organic phases were combined, concentrated under reduced pressure, and then slowly cooled to separate out the product. 4.55g of a white product with a purity of 98.62%, an optical purity of 99.66%, and a total yield of 91% was obtained.

### Example 5

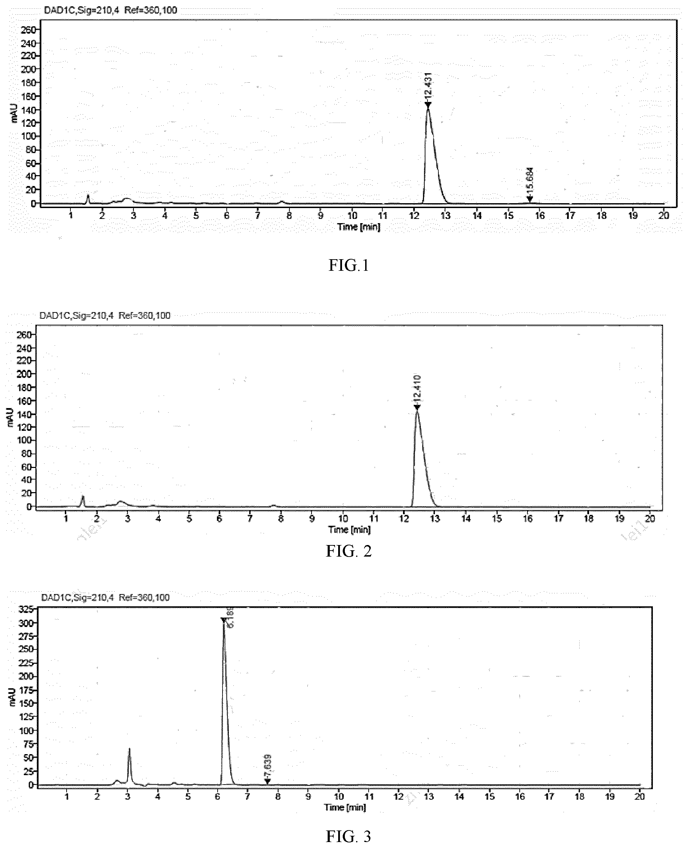

7.5g of methyl β-carbonyltetradecanoate and 11.25g of glucose were weighed and placed in a 100mL three-necked flask, and then added with 50mL of PBS buffer with a concentration of 50mM and a pH value of 7.0. The three-necked flask was put into a constant temperature water bath, in which the stirring speed was adjusted to 800rpm, and the temperature was 35°C, and then 150mg NADP⁺, 200mg glucose dehydrogenase GDH enzyme powder (shown in SEQ ID NO:3), and 750mg ketoreductase JR3789 enzyme powder (shown in SEQ ID NO: 1) were added respectively to obtain a mixed reaction solution. The pH was adjusted and maintained at 7.0-7.5 using 2M NaOH solution, and the temperature was maintained at 35°C. The reaction progress was monitored by HPLC. The reaction was completed in 15h, and the conversion rate was measured as >99%. The test results are shown in Table 1 below, and the HPLC chromatogram is shown in Figure 1.

After the reaction was completed, the obtained solution was heated to a temperature of 60°C and maintained for 15min, then cooled to 20-25°C, and added with 100mL of ethyl acetate for extraction with stirring for 20min, and filtered. The organic phase was taken after the filtrate was layered. The aqueous phase was extracted once more with 80mL of ethyl acetate, the layers were separated, and the organic phase was taken. The organic phases were combined, concentrated under reduced pressure, and then slowly cooled to separate out the product. A crude product with a purity of 99.60% and an ee value of 98.68% was obtained. The crude product was added with 2 times the volume of n-hexane, heated, dissolved, cooled for crystalization, filtered, and air-dried at room temperature to obtain 6.53g of a white crystal product with a measured purity of 99.99%, an ee value of 99.86%, and a total yield of 87%. The purity data of the tested product is shown in Table 2, the HPLC chromatogram is shown in Figure 2, the chiral purity data of the product is shown in Table 3, and the HPLC chromatogram is shown in Figure 3.

**Table 1 Data sheet of measured conversion rates**

| RT (min) | Type | Width (min) | Area | Height | Area% |
|---|---|---|---|---|---|
| 12.431 | BB | 1.86 | 3001.36 | 142.52 | 99.06 |
| 15.684 | BBA | 0.79 | 28.40 | 1.48 | 0.94 |
| | | Sum | 3029.76 | | |

**Table 2 Data sheet of measured purity of the product**

| RT (min) | Type | Width (min) | Area | Height | Area% |
|---|---|---|---|---|---|
| 12.410 | BB | 1.36 | 3062.49 | 145.21 | 100.00 |
| | | Sum | 3062.49 | | |

**Table 3 Data sheet of measured chiral purity of the product**

| RT (min) | Type | Width (min) | Area | Height | Area% |
|---|---|---|---|---|---|
| 6.189 | BBA | 0.52 | 2944.36 | 298.07 | 99.93 |
| 7.639 | BB | 0.30 | 2.00 | 0.23 | 0.07 |
| | | Sum | 2946.336 | | |

### Example 6

### (1) Construction of fusion enzyme G3790 expression plasmid

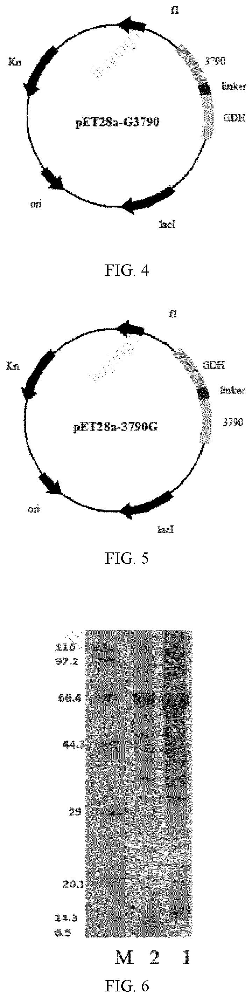

The gene sequence of linker (SEQ ID NO:5) was inserted at the 3' end of the gene fragment of glucose dehydrogenase GDH (SEQ ID NO:3), and the gene fragment of ketoreductase JR3790 (SEQ ID NO:4) was connected to constitute the fusion enzyme G3790 sequence (SEQ ID NO: 6) (synthesized by Nanjing GenScript Biotechnology Co., Ltd.), which was further connected to the carrier pET28a through the restriction sites NdeI and XhoI at both ends to constitute a double-enzyme fusion expression plasmid pET28a-G3790. The plasmid map of the recombinant plasmid is as shown in Figure 4.

### (2) Construction of fusion enzyme 3790G expression plasmid

The gene sequence of linker (SEQ ID NO:5) was inserted at the 3' end of the gene fragment of ketoreductase JR3790 (SEQ ID NO:4), and the gene fragment of glucose dehydrogenase GDH (SEQ ID NO:3) was connected to constitute the fusion enzyme sequence (SEQ ID NO: 7) (synthesized by Nanjing GenScript Biotechnology Co., Ltd.), which was further connected to the carrier pET28a through the restriction sites NdeI and XhoI at both ends to constitute a double-enzyme fusion expression plasmid pET28a-3790G. The plasmid map of the recombinant plasmid is as shown in Figure 5.

### (3) Preparation of fusion enzyme G3790 and fusion enzyme 3790G

The constructed fusion enzyme expression plasmids pET28a-G3790 and pET28a-3790G were transferred into Escherichia coli competent E.coli BL21 (DE3) strains. The positive clone transformants were obtained by screening. The single clone containing the recombinant plasmid was selected and inoculated into a test tube containing 5ml LB medium (100 µg/ml kanamycin). After overnight incubation at 37°C and 200rpm, the bacterial solution was transferred to a conical flask containing 1L of LB medium at an inoculum of 2%, and incubated at 37°C and 200rpm until the OD600 reached about 0.6. The bacterial solution was added with isopropyl-β-D-thiogalactoside (IPTG) inducer (final concentration 0.3mM), and cultured continuously at 25°C for 12h. The bacterial cells were collected by centrifugation. The bacterial cells were diluted with PBS (pH=7.0), resuspended and sonicated for disruption, and centrifuged to obtain the supernatant, i.e. the crude enzyme solution.

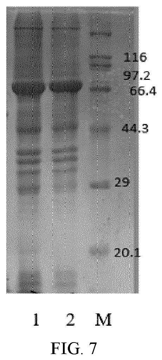

The crude enzyme solution obtained by expression was subjected to SDS-polyacrylamide gel electrophoresis (SDS-PAGE) to identify protein bands. Figure 6 shows the protein electrophoresis of G3790 fusion enzyme, wherein lane M is the protein Marker (GenScript), and lane 1 is the total protein after whole cell disruption, and lane 2 is the centrifugation supernatant of whole cell disruption, wherein the theoretical molecular weight of the fusion enzyme should be 67kDa, and the amino acid sequence thereof is as shown in SEQ ID NO:8. Figure 7 shows the protein electrophoresis of 3790G fusion enzyme, wherein lane M is the protein Marker (GenScript), and lane 1 is the total protein after whole cell disruption, and lane 2 is the centrifugation supernatant of whole cell disruption. The theoretical molecular weights of the two fusion enzymes are both 67kDa, and the amino acid sequences thereof are shown in SEQ ID NO:9. The two fusion enzymes are both soluble proteins, and their molecular weights are close to the corresponding theoretical molecular weights.

The crude enzyme solution was pre-frozen overnight and freeze-dried for 24h-36h to obtain fusion enzyme G3790 enzyme powder and fusion enzyme 3790G enzyme powder.

### (4) Preparation of the orlistat intermediate

7.5kg of methyl β-carbonyltetradecanoate and 9.375kg of glucose were weighed and placed in a 100mL glass reactor, and then added with 25L of PBS buffer with a concentration of 50mM and a pH value of 7.0. The high and low temperature cycle integrated machine maintained the reactor temperature at 35°C, and mechanical stirring speed was adjusted to 180rpm. Then 18g NADP⁺and 800g 3790G enzyme powder were added respectively to obtain a mixed reaction solution. The pH was adjusted and maintained at 7.0-7.5 using 2M NaOH solution. The reaction progress was monitored by HPLC. The reaction was completed in 13h, and the conversion rate was measured as >99%.

After the reaction was completed, the obtained solution was heated to a temperature of 60°C and maintained for 15min, then cooled to 20-25°C, and filtered by suction to collect the filter cake. The filter cake was added with 15L of ethyl acetate. The mixture was extracted and stirred for 20min, and filtered by suction to collect the organic phase. The organic phases were combined, concentrated under reduced pressure, and then slowly cooled to separate out the product. A crude product with a purity of 98.5% and an ee value of 98.6% was obtained. The crude product was added with 2 times the volume of n-hexane, heated, dissolved, cooled for crystalization, filtered, and air-dried at room temperature to obtain 6.45 kg of a white crystal product with a measured purity of 99.70%, an ee value of 99.88%, and a total yield of 86%.

## Claims

1. Use of a biological enzyme in the preparation of an orlistat intermediate, wherein the substrate of the biological enzyme is β-carbonyltetradecanoate, and the structural formula is as shown in I, and the orlistat intermediate is (R)-β-hydroxytetradecanoate, and the structural formula is as shown in II, and R in structural formula I refers to a saturated alkyl group containing 1-3 carbon atoms, and R in structural formula II refers to a saturated alkyl group containing 1-3 carbon atoms; 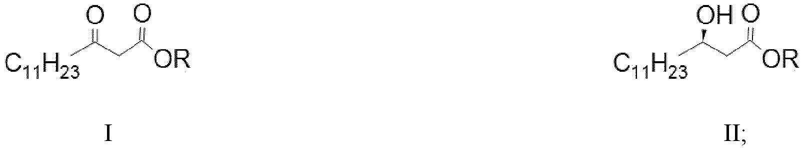 the biological enzyme is ketoreductase or a fusion enzyme of ketoreductase and glucose dehydrogenase, and the amino acid sequence of the ketoreductase is as shown in SEQ ID NO: 1 and/or as shown in SEQ ID NO: 2, and/or as shown in SEQ ID NO:4; the amino acid sequence of the fusion enzyme of ketoreductase and glucose dehydrogenase is as shown in SEQ ID NO:8 and/or SEQ ID NO:9.

2. The use according to claim 1, wherein the biological enzyme is a fusion enzyme of ketoreductase and glucose dehydrogenase, and in the fusion enzyme, the ketoreductase and the glucose dehydrogenase are connected by a linker; optionally, the amino acid sequence of the linker is as shown in SEQ ID NO:5; and/or
wherein the biological enzyme is a fusion enzyme of ketoreductase and glucose dehydrogenase, which is ketoreductase-linker-glucose dehydrogenase, or glucose dehydrogenase- linker-ketoreductase.

3. The use according to any one of claims 1-2, wherein the amino acid sequence of the glucose dehydrogenase is as shown in SEQ ID NO:3; and/or
wherein the biological enzyme is enzyme powder and/or enzyme solution and/or immobilized enzyme; and/or
wherein R in the structural formula I is any one of methyl, ethyl, n-propyl or isopropyl, and R in the structural formula II is any one of methyl, ethyl, n-propyl or isopropyl.

4. A fusion enzyme, wherein the fusion enzyme is a fusion enzyme of ketoreductase and glucose dehydrogenase; the amino acid sequence of the fusion enzyme is as shown in SEQ ID NO:8 and/or SEQ ID NO:9.

5. The fusion enzyme according to claim 4, wherein in the fusion enzyme, the ketoreductase and the glucose dehydrogenase are connected by a linker; optionally, the amino acid sequence of the linker is as shown in SEQ ID NO:5.

6. The fusion enzyme according to claim 4 or 5, wherein the fusion enzyme is ketoreductase-linker-glucose dehydrogenase, or glucose dehydrogenase-linker-ketoreductase;
optionally, the amino acid sequence of the ketoreductase is as shown in SEQ ID NO: 1 and/or as shown in SEQ ID NO: 2, and/or as shown in SEQ ID NO: 4;
optionally, the amino acid sequence of the glucose dehydrogenase is as shown in SEQ ID NO:3.

7. A nucleotide sequence for encoding the fusion enzyme of any one of claims 4-6, wherein the nucleotide sequence of the fusion enzyme is as shown in SEQ ID NO: 6 or SEQ ID NO: 7.

8. A method for constructing the nucleotide sequence according to claim 7, comprising: inserting the linker sequence shown in SEQ ID NO:5 at the 3' end of the gene fragment of glucose dehydrogenase shown in SEQ ID NO:3, and then connecting the gene fragment of the ketoreductase shown in SEQ ID NO:4 to form a recombinant plasmid with the nucleotide sequence shown in SEQ ID NO:6; and/or
inserting the linker sequence shown in SEQ ID NO:5 at the 3' end of the gene fragment of the ketoreductase shown in SEQ ID NO:4, and then connecting the gene fragment of glucose dehydrogenase shown in SEQ ID NO:3 to form the nucleotide sequence shown in SEQ ID NO:7.

9. A composition containing a biological enzyme and a substrate, wherein the substrate is β-carbonyltetradecanoate, and its structural formula is as shown in formula I; the biological enzyme is ketoreductase or a fusion enzyme of ketoreductase and glucose dehydrogenase, and the amino acid sequence of the ketoreductase is as shown in SEQ ID NO: 1 and/or as shown in SEQ ID NO:2, and/or as shown in SEQ ID NO:4; the amino acid sequence of the fusion enzyme of ketoreductase and glucose dehydrogenase is as shown in SEQ ID NO:8 and/or as shown in SEQ ID NO:9; 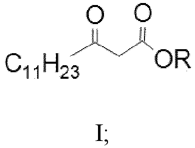 R in the structural formula I refers to a saturated alkyl group containing 1-3 carbon atoms.

10. The composition according to claim 9, wherein the weight ratio of the biological enzyme to the substrate is 1:1.1-150; preferably 1:20, 1:30, 1:40, 1:50, 1:60, 1 : 70, 1:80, 1:90, 1:100, 1:110, 1:120, 1:130, 1:140 and/or 1:150.

11. A reaction system, wherein the reaction system is composed of the substrate shown in I, biological enzyme, glucose, glucose dehydrogenase, NADP⁺ and buffer solution; the biological enzyme described here is ketoreductase, and the amino acid sequence of the ketoreductase is as shown in SEQ ID NO: 1 and/or as shown in SEQ ID NO:2, and/or as shown in SEQ ID NO:4; or the reaction system is composed of the substrate shown in I, biological enzyme, glucose, NADP⁺ and buffer solution; wherein, the biological enzyme is a fusion enzyme of ketoreductase and glucose dehydrogenase, and the amino acid sequence of the ketoreductase is as shown in SEQ ID NO: 1 and/or as shown in SEQ ID NO: 2, and/or as shown in SEQ ID NO: 4; the amino acid sequence of the fusion enzyme of ketoreductase and glucose dehydrogenase is as shown in SEQ ID NO:8 and/or as shown in SEQ ID NO:9; 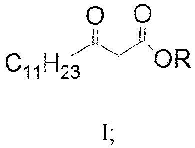 R in the structural formula I refers to a saturated alkyl group containing 1-3 carbon atoms.

12. The reaction system according to claim 11, wherein the pH value of the reaction system is 6.0-8.0; optionally, the buffer solution is a PBS buffer or a Tris-HCl buffer; and/or
wherein the concentration of the substrate is 20g/L-150g/L; and/or
wherein the concentration of the NADP⁺ is 0.1-0.5 g/L; and/or
wherein the molar ratio of compound I to the glucose is 1: 1.2-4; and/or
wherein the concentration of the buffer is 0.01-0.5mol/L; and/or
wherein the reaction time of the reaction system does not exceed 15 hours, and the reaction solution is obtained.

13. A method for preparing a orlistat intermediate, comprising reacting the reaction system according to any one of claims 11 to 12, wherein the reaction system is reacted at 20-40°C with stirring to obtain the reaction solution of the orlistat intermediate, and the intermediate of the orlistat intermediate is (R)-β-hydroxytetradecanoate, and the structural formula is as shown in II; 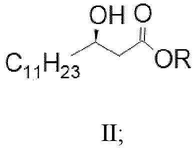 R in the structural formula II refers to a saturated alkyl group containing 1-3 carbon atoms.

14. The method according to claim 13, wherein an aqueous NaOH solution is used as the pH adjusting agent; and/or
wherein the reaction time does not exceed 15 hours; and/or
wherein the orlistat intermediate is extracted with a solvent from the obtained reaction solution.

15. The method according to claim 14, wherein the solvent is anhydrous ethanol or ethyl acetate; and/or
wherein the obtained extract is concentrated under reduced pressure, and cooled for crystallization, to obtain a white crystalline solid, namely compound II.

## Patentansprüche

1. Verwendung eines biologischen Enzyms bei der Herstellung eines Orlistat-Zwischenprodukts, wobei das Substrat des biologischen Enzyms β-Carbonyltetradecanoat ist und die Strukturformel wie in I gezeigt ist, und das Orlistat-Zwischenprodukt (R)-β-Hydroxytetradecanoat ist und die Strukturformel wie in II gezeigt ist, und R in der Strukturformel I sich auf eine gesättigte Alkylgruppe mit 1-3 Kohlenstoffatomen bezieht, und R in der Strukturformel II sich auf eine gesättigte Alkylgruppe mit 1-3 Kohlenstoffatomen bezieht; 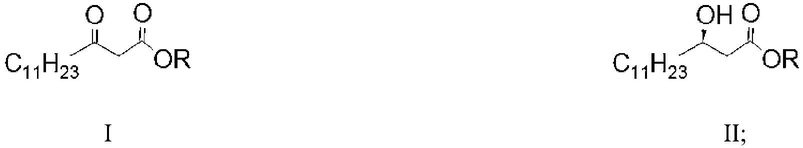 das biologische Enzym eine Ketoreduktase oder ein Fusionsenzym aus Ketoreduktase und Glucosedehydrogenase ist, und die Aminosäuresequenz der Ketoreduktase wie in SEQ ID NO: 1 und/oder wie in SEQ ID NO: 2 und/oder wie in SEQ ID NO: 4 gezeigt ist; die Aminosäuresequenz des Fusionsenzyms aus Ketoreduktase und Glucosedehydrogenase wie in SEQ ID NO: 8 und/oder SEQ ID NO: 9 gezeigt ist.

2. Verwendung nach Anspruch 1, wobei das biologische Enzym ein Fusionsenzym aus Ketoreduktase und Glucosedehydrogenase ist, und in dem Fusionsenzym die Ketoreduktase und die Glucosedehydrogenase durch einen Linker verbunden sind; gegebenenfalls die Aminosäuresequenz des Linkers wie in SEQ ID NO: 5 gezeigt ist; und/oder
wobei das biologische Enzym ein Fusionsenzym aus Ketoreduktase und Glucosedehydrogenase ist, das Ketoreduktase-Linker-Glucosedehydrogenase oder Glucosedehydrogenase-Linker-Ketoreduktase ist.

3. Verwendung nach einem der Ansprüche 1-2, wobei die Aminosäuresequenz der Glucosedehydrogenase wie in SEQ ID NO: 3 gezeigt ist; und/oder
wobei das biologische Enzym Enzympulver und/oder Enzymlösung und/oder immobilisiertes Enzym ist; und/oder
wobei R in der Strukturformel I eines von Methyl, Ethyl, n-Propyl oder Isopropyl ist, und R in der Strukturformel II eines von Methyl, Ethyl, n-Propyl oder Isopropyl ist.

4. Fusionsenzym, wobei das Fusionsenzym ein Fusionsenzym aus Ketoreduktase und Glucosedehydrogenase ist; die Aminosäuresequenz des Fusionsenzyms wie in SEQ ID NO: 8 und/oder SEQ ID NO: 9 gezeigt ist.

5. Fusionsenzym nach Anspruch 4, wobei in dem Fusionsenzym die Ketoreduktase und die Glucosedehydrogenase durch einen Linker verbunden sind; gegebenenfalls die Aminosäuresequenz des Linkers wie in SEQ ID NO: 5 gezeigt ist.

6. Fusionsenzym nach Anspruch 4 oder 5, wobei das Fusionsenzym Ketoreduktase-Linker-Glucosedehydrogenase oder Glucosedehydrogenase-Linker-Ketoreduktase ist;
gegebenenfalls die Aminosäuresequenz der Ketoreduktase wie in SEQ ID NO: 1 und/oder wie in SEQ ID NO: 2 und/oder wie in SEQ ID NO: 4 gezeigt ist;
gegebenenfalls die Aminosäuresequenz der Glucosedehydrogenase wie in SEQ ID NO: 3 gezeigt ist.

7. Nukleotidsequenz zur Codierung des Fusionsenzyms nach einem der Ansprüche 4-6, wobei die Nukleotidsequenz des Fusionsenzyms wie in SEQ ID NO: 6 oder SEQ ID NO: 7 gezeigt ist.

8. Verfahren zur Konstruktion der Nukleotidsequenz nach Anspruch 7, umfassend: Einfügen der in SEQ ID NO: 5 gezeigten Linkersequenz am 3'-Ende des in SEQ ID NO: 3 gezeigten Genfragments der Glucosedehydrogenase, und anschließendes Verbinden des in SEQ ID NO: 4 gezeigten Genfragments der Ketoreduktase, um ein rekombinantes Plasmid mit der in SEQ ID NO: 6 gezeigten Nukleotidsequenz zu bilden; und/oder
Einfügen der in SEQ ID NO: 5 gezeigten Linkersequenz am 3'-Ende des in SEQ ID NO: 4 gezeigten Genfragments der Ketoreduktase, und anschließendes Verbinden des in SEQ ID NO: 3 gezeigten Genfragments der Glucosedehydrogenase, um die in SEQ ID NO: 7 gezeigte Nukleotidsequenz zu bilden.

9. Zusammensetzung, enthaltend ein biologisches Enzym und ein Substrat, wobei das Substrat β-Carbonyltetradecanoat ist und seine Strukturformel wie in Formel I gezeigt ist; das biologische Enzym eine Ketoreduktase oder ein Fusionsenzym aus Ketoreduktase und Glucosedehydrogenase ist, und die Aminosäuresequenz der Ketoreduktase wie in SEQ ID NO: 1 und/oder wie in SEQ ID NO: 2 und/oder wie in SEQ ID NO: 4 gezeigt ist; die Aminosäuresequenz des Fusionsenzyms aus Ketoreduktase und Glucosedehydrogenase wie in SEQ ID NO: 8 und/oder wie in SEQ ID NO: 9 gezeigt ist; 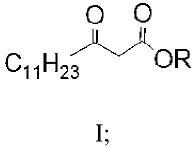 R in der Strukturformel I sich auf eine gesättigte Alkylgruppe mit 1-3 Kohlenstoffatomen bezieht.

10. Zusammensetzung nach Anspruch 9, wobei das Gewichtsverhältnis des biologischen Enzyms zum Substrat 1:1,1-150 beträgt; vorzugsweise 1:20, 1:30, 1:40, 1:50, 1:60, 1:70, 1:80, 1:90, 1:100, 1:110, 1:120, 1:130, 1:140 und/oder 1:150.

11. Reaktionssystem, wobei das Reaktionssystem aus dem in I gezeigten Substrat, biologischem Enzym, Glucose, Glucosedehydrogenase, NADP+ und Pufferlösung besteht; das hier beschriebene biologische Enzym eine Ketoreduktase ist, und die Aminosäuresequenz der Ketoreduktase wie in SEQ ID NO: 1 und/oder wie in SEQ ID NO: 2 und/oder wie in SEQ ID NO: 4 gezeigt ist; oder das Reaktionssystem aus dem in I gezeigten Substrat, biologischem Enzym, Glucose, NADP+ und Pufferlösung besteht; wobei das biologische Enzym ein Fusionsenzym aus Ketoreduktase und Glucosedehydrogenase ist, und die Aminosäuresequenz der Ketoreduktase wie in SEQ ID NO: 1 und/oder wie in SEQ ID NO: 2 und/oder wie in SEQ ID NO: 4 gezeigt ist; die Aminosäuresequenz des Fusionsenzyms aus Ketoreduktase und Glucosedehydrogenase wie in SEQ ID NO: 8 und/oder wie in SEQ ID NO: 9 gezeigt ist; 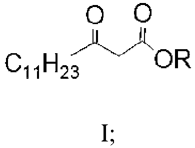 R in der Strukturformel I sich auf eine gesättigte Alkylgruppe mit 1-3 Kohlenstoffatomen bezieht.

12. Reaktionssystem nach Anspruch 11, wobei der pH-Wert des Reaktionssystems 6,0-8,0 beträgt; gegebenenfalls die Pufferlösung ein PBS-Puffer oder ein Tris-HCl-Puffer ist; und/oder
wobei die Konzentration des Substrats 20 g/L-150 g/L beträgt; und/oder
wobei die Konzentration des NADP+ 0,1-0,5 g/L beträgt; und/oder
wobei das Molverhältnis der Verbindung I zur Glucose 1:1,2-4 beträgt; und/oder
wobei die Konzentration des Puffers 0,01-0,5 mol/L beträgt; und/oder
wobei die Reaktionszeit des Reaktionssystems 15 Stunden nicht überschreitet, und die Reaktionslösung erhalten wird.

13. Verfahren zur Herstellung eines Orlistat-Zwischenprodukts, umfassend das Umsetzen des Reaktionssystems nach einem der Ansprüche 11 bis 12, wobei das Reaktionssystem bei 20-40 °C unter Rühren umgesetzt wird, um die Reaktionslösung des Orlistat-Zwischenprodukts zu erhalten, und das Zwischenprodukt des Orlistat-Zwischenprodukts (R)-β-Hydroxytetradecanoat ist, und die Strukturformel wie in II gezeigt ist; 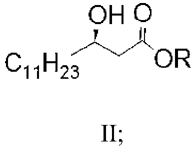 R in der Strukturformel II sich auf eine gesättigte Alkylgruppe mit 1-3 Kohlenstoffatomen bezieht.

14. erfahren nach Anspruch 13, wobei eine wässrige NaOH-Lösung als pH-Einstellmittel verwendet wird; und/oder
wobei die Reaktionszeit 15 Stunden nicht überschreitet; und/oder
wobei das Orlistat-Zwischenprodukt mit einem Lösungsmittel aus der erhaltenen Reaktionslösung extrahiert wird.

15. Verfahren nach Anspruch 14, wobei das Lösungsmittel wasserfreies Ethanol oder Ethylacetat ist; und/oder
wobei der erhaltene Extrakt unter vermindertem Druck konzentriert und zur Kristallisation gekühlt wird, um einen weißen kristallinen Feststoff, nämlich die Verbindung II, zu erhalten.

## Revendications

1. Utilisation d'une enzyme biologique dans la préparation d'un intermédiaire d'orlistat, dans laquelle le substrat de l'enzyme biologique est le β-carbonyltétradécanoate, et la formule structurale est telle que représentée en I, et l'intermédiaire d'orlistat est le (R)-β-hydroxytétradécanoate, et la formule structurale est telle que représentée en II, et R dans la formule structurale I désigne un groupe alkyle saturé contenant 1 à 3 atomes de carbone, et R dans la formule structurale II désigne un groupe alkyle saturé contenant 1 à 3 atomes de carbone; 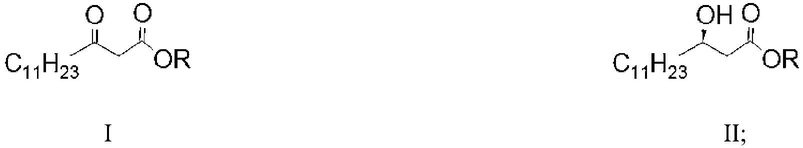 l'enzyme biologique est une cétoréductase ou une enzyme de fusion de cétoréductase et de glucose déshydrogénase, et la séquence d'acides aminés de la cétoréductase est telle que représentée dans SEQ ID NO : 1 et/ou telle que représentée dans SEQ ID NO : 2, et/ou telle que représentée dans SEQ ID NO : 4 ; la séquence d'acides aminés de l'enzyme de fusion de cétoréductase et de glucose déshydrogénase est telle que représentée dans SEQ ID NO : 8 et/ou SEQ ID NO : 9.

2. Utilisation selon la revendication 1, dans laquelle l'enzyme biologique est une enzyme de fusion de cétoréductase et de glucose déshydrogénase, et dans l'enzyme de fusion, la cétoréductase et la glucose déshydrogénase sont reliées par un lieur ; éventuellement, la séquence d'acides aminés du lieur est telle que représentée dans SEQ ID NO : 5 ; et/ou
dans laquelle l'enzyme biologique est une enzyme de fusion de cétoréductase et de glucose déshydrogénase, qui est cétoréductase-lieur-glucose déshydrogénase, ou glucose déshydrogénase-lieur-cétoréductase.

3. Utilisation selon l'une quelconque des revendications 1 à 2, dans laquelle la séquence d'acides aminés de la glucose déshydrogénase est telle que représentée dans SEQ ID NO : 3 ; et/ou
dans laquelle l'enzyme biologique est une poudre d'enzyme et/ou une solution d'enzyme et/ou une enzyme immobilisée ; et/ou
dans laquelle R dans la formule structurale I est l'un quelconque parmi méthyle, éthyle, n-propyle ou isopropyle, et R dans la formule structurale II est l'un quelconque parmi méthyle, éthyle, n-propyle ou isopropyle.

4. Enzyme de fusion, dans laquelle l'enzyme de fusion est une enzyme de fusion de cétoréductase et de glucose déshydrogénase ; la séquence d'acides aminés de l'enzyme de fusion est telle que représentée dans SEQ ID NO : 8 et/ou SEQ ID NO : 9.

5. Enzyme de fusion selon la revendication 4, dans laquelle, dans l'enzyme de fusion, la cétoréductase et la glucose déshydrogénase sont reliées par un lieur ; éventuellement, la séquence d'acides aminés du lieur est telle que représentée dans SEQ ID NO : 5.

6. Enzyme de fusion selon la revendication 4 ou 5, dans laquelle l'enzyme de fusion est cétoréductase-lieur-glucose déshydrogénase, ou glucose déshydrogénase-lieur-cétoréductase ;
éventuellement, la séquence d'acides aminés de la cétoréductase est telle que représentée dans SEQ ID NO : 1 et/ou telle que représentée dans SEQ ID NO : 2, et/ou telle que représentée dans SEQ ID NO : 4 ;
éventuellement, la séquence d'acides aminés de la glucose déshydrogénase est telle que représentée dans SEQ ID NO : 3.

7. Séquence nucléotidique pour coder l'enzyme de fusion selon l'une quelconque des revendications 4 à 6, dans laquelle la séquence nucléotidique de l'enzyme de fusion est telle que représentée dans SEQ ID NO : 6 ou SEQ ID NO : 7.

8. Procédé de construction de la séquence nucléotidique selon la revendication 7, comprenant : l'insertion de la séquence de lieur représentée dans SEQ ID NO : 5 à l'extrémité 3' du fragment de gène de la glucose déshydrogénase représenté dans SEQ ID NO : 3, puis le raccordement du fragment de gène de la cétoréductase représenté dans SEQ ID NO : 4 pour former un plasmide recombinant avec la séquence nucléotidique représentée dans SEQ ID NO : 6 ; et/ou
l'insertion de la séquence de lieur représentée dans SEQ ID NO : 5 à l'extrémité 3' du fragment de gène de la cétoréductase représenté dans SEQ ID NO : 4, puis le raccordement du fragment de gène de la glucose déshydrogénase représenté dans SEQ ID NO : 3 pour former la séquence nucléotidique représentée dans SEQ ID NO : 7.

9. Composition contenant une enzyme biologique et un substrat, dans laquelle le substrat est le β-carbonyltétradécanoate, et sa formule structurale est telle que représentée dans la formule I ; l'enzyme biologique est une cétoréductase ou une enzyme de fusion de cétoréductase et de glucose déshydrogénase, et la séquence d'acides aminés de la cétoréductase est telle que représentée dans SEQ ID NO : 1 et/ou telle que représentée dans SEQ ID NO : 2, et/ou telle que représentée dans SEQ ID NO : 4 ; la séquence d'acides aminés de l'enzyme de fusion de cétoréductase et de glucose déshydrogénase est telle que représentée dans SEQ ID NO : 8 et/ou telle que représentée dans SEQ ID NO : 9; 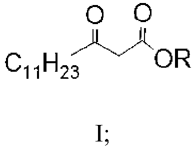 R dans la formule structurale I désigne un groupe alkyle saturé contenant 1 à 3 atomes de carbone.

10. Composition selon la revendication 9, dans laquelle le rapport en poids de l'enzyme biologique au substrat est de 1:1,1-150 ; de préférence 1:20, 1:30, 1:40, 1:50, 1:60, 1:70, 1:80, 1:90, 1:100, 1:110, 1:120, 1:130, 1:140 et/ou 1:150.

11. Système réactionnel, dans lequel le système réactionnel est composé du substrat représenté en I, d'une enzyme biologique, de glucose, de glucose déshydrogénase, de NADP+ et d'une solution tampon ; l'enzyme biologique décrite ici est une cétoréductase, et la séquence d'acides aminés de la cétoréductase est telle que représentée dans SEQ ID NO : 1 et/ou telle que représentée dans SEQ ID NO : 2, et/ou telle que représentée dans SEQ ID NO : 4 ; ou le système réactionnel est composé du substrat représenté en I, d'une enzyme biologique, de glucose, de NADP+ et d'une solution tampon ; dans lequel l'enzyme biologique est une enzyme de fusion de cétoréductase et de glucose déshydrogénase, et la séquence d'acides aminés de la cétoréductase est telle que représentée dans SEQ ID NO : 1 et/ou telle que représentée dans SEQ ID NO : 2, et/ou telle que représentée dans SEQ ID NO : 4 ; la séquence d'acides aminés de l'enzyme de fusion de cétoréductase et de glucose déshydrogénase est telle que représentée dans SEQ ID NO : 8 et/ou telle que représentée dans SEQ ID NO : 9; 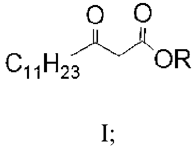 R dans la formule structurale I désigne un groupe alkyle saturé contenant 1 à 3 atomes de carbone.

12. Système réactionnel selon la revendication 11, dans lequel la valeur du pH du système réactionnel est de 6,0 à 8,0 ; éventuellement, la solution tampon est un tampon PBS ou un tampon Tris-HCl ; et/ou
dans lequel la concentration du substrat est de 20 g/L à 150 g/L ; et/ou
dans lequel la concentration du NADP+ est de 0,1 à 0,5 g/L ; et/ou
dans lequel le rapport molaire du composé I au glucose est de 1:1,2-4 ; et/ou
dans lequel la concentration du tampon est de 0,01 à 0,5 mol/L ; et/ou
dans lequel le temps de réaction du système réactionnel ne dépasse pas 15 heures, et la solution réactionnelle est obtenue.

13. Procédé de préparation d'un intermédiaire d'orlistat, comprenant la mise en réaction du système réactionnel selon l'une quelconque des revendications 11 à 12, dans lequel le système réactionnel est mis à réagir à 20-40 °C sous agitation pour obtenir la solution réactionnelle de l'intermédiaire d'orlistat, et l'intermédiaire de l'intermédiaire d'orlistat est le (R)-β-hydroxytétradécanoate, et la formule structurale est telle que représentée en II ; 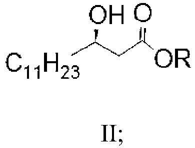 R dans la formule structurale II désigne un groupe alkyle saturé contenant 1 à 3 atomes de carbone.

14. Procédé selon la revendication 13, dans lequel une solution aqueuse de NaOH est utilisée comme agent d'ajustement du pH ; et/ou
dans lequel le temps de réaction ne dépasse pas 15 heures ; et/ou
dans lequel l'intermédiaire d'orlistat est extrait avec un solvant à partir de la solution réactionnelle obtenue.

15. Procédé selon la revendication 14, dans lequel le solvant est de l'éthanol anhydre ou de l'acétate d'éthyle ; et/ou
dans lequel l'extrait obtenu est concentré sous pression réduite, et refroidi pour la cristallisation, afin d'obtenir un solide cristallin blanc, à savoir le composé II.
